(19) Europäisches Patentamt
European Patent Office
Office européen des brevets

(11) **EP 1 962 962 B1**

(12) **EUROPÄISCHE PATENTSCHRIFT**

(45) Veröffentlichungstag und Bekanntmachung des Hinweises auf die Patenterteilung:
**02.09.2015 Patentblatt 2015/36**

(51) Int Cl.:
***A61Q 5/06*** (2006.01)     ***A61K 8/02*** (2006.01)
***A61K 8/72*** (2006.01)

(21) Anmeldenummer: **06829501.3**

(22) Anmeldetag: **11.12.2006**

(86) Internationale Anmeldenummer:
**PCT/EP2006/011913**

(87) Internationale Veröffentlichungsnummer:
**WO 2007/073857 (05.07.2007 Gazette 2007/27)**

(54) **PULVERFÖRMIGE STYLINGMITTEL UND DEREN SPENDERSYSTEME**

POWDERY STYLING AGENTS AND THE DISPENSER SYSTEMS THEREOF

PRODUITS COIFFANTS SOUS FORME DE POUDRE ET LEURS SYSTÈMES DISTRIBUTEURS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.12.2005 DE 102005062268**

(43) Veröffentlichungstag der Anmeldung:
**03.09.2008 Patentblatt 2008/36**

(73) Patentinhaber: **Henkel AG & Co. KGaA
40589 Düsseldorf (DE)**

(72) Erfinder:
• **KAPLAN, Anett
40235 Düsseldorf (DE)**

• **RICHTERS, Bernd
21129 Hamburg (DE)**

(56) Entgegenhaltungen:
-A-                             DE-A- 10 312 270
DE-U1-202004 015 369    US-A- 786 125
US-A- 4 630 954            US-A1- 2002 014 246
US-A1- 2003 161 675      US-A1- 2004 262 340

• **"Grunella-Seifenmuele-Seifenspender", ,
Retrieved from the Internet: URL:http:
//www.ebay.de/itm/Grunella-Seifen muele-
Seifenspender-absolut-kultig-soap-mi II/
280870574442 [retrieved on 2012-11-21]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft Mittel zum Festigen und Frisieren keratinischer Fasern, insbesondere menschlicher Haare aus einem geeigneten Spendersystem heraus in Form eines feinen Pulvers, welches gegebenenfalls erst unmittelbar vor der Anwendung frisch gemahlen, gehobelt oder geraspelt wird, sowie die Verwendung dieser Zubereitung zur Behandlung keratinischer Fasern, insbesondere menschlicher Haare.

Kosmetische Mittel zur Pflege und Erhalt der natürlichen Funktionen von Haut und Haar gewinnen mehr und mehr an Bedeutung. Dazu tragen unter anderem die veränderten Verbrauchergewohnheiten und Modetrends bei. So werden beispielsweise durch das intensive Nutzen von Sonnenstudios Haut und Haar in ihrer Struktur stärker durch UV-Licht nachhaltig beeinträchtigt. Diese Beeinträchtigungen zeigen sich auf der Haut wie dem Haar beispielsweise durch einen Verlust der Elastizität.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen.

Keratinische Fasern, insbesondere menschliche Haare, werden heutzutage einer Vielzahl von Behandlungen unterzogen. Dabei spielen die Behandlungen, die zu einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Fönwellen etc. erzielt werden.

Üblicherweise werden beispielsweise Haarsprays, Pumpsprühfestiger, Haargele oder Haarwachse als einphasige Produkte in blickdichten Verpackungen angeboten. Es hat sich in der letzten Zeit jedoch gezeigt, dass der Verbraucher neben den Ansprüchen an den Halt und die Pflege auch immer höhere Anforderungen an das Design und die einfache Handhabbarkeit der Produkte stellt, so dass nun verstärkt Produkte in transparenten Verpackungen hergestellt werden, die dem Verbraucher die Erkennbarkeit des Füllstandes erleichtern und den Produkten eine größere Attraktivität verleihen.

Es besteht aber weiterhin die Aufgabe, entsprechende Mittel zu entwickeln, die hinsichtlich der anwendungstechnischen Eigenschaften, beispielsweise dem Halt, der Fülle, der Möglichkeit des Formens von Frisuren und der Trocknungszeit bei festigenden Haarpflegemitteln die vom Verbraucher gesteckten Erwartungen erfüllen und einfach und leicht portionierbar sind, ,die einfach und leicht verpackt sind, so daß sie jederzeit überall hin mitgeführt werden können und somit jederzeit überall anwendbar sind oder jederzeit bereits an beliebigen Orten öffentlich zur Verfügung stehen.

So ersetzten in der Vergangenheit beispielsweise Mittel auf wäßriger Basis Mittel auf der Basis von flüchtigen organischen Verbindungen. Dabei entstand das Problem der geringeren Flüchtigkeit von Wasser im Vergleich zu den Alkoholen, was sich in längeren Trocknungszeiten auf dem Haar niederschlägt. Weiterhin ist aufgrund der häufig schlechteren Löslichkeit polymerer Verbindungen in wäßrigen Systemen diese Umstellung auch häufig mit dem Nachteil verbunden, daß beim Aufbringen der gewünschten Polymermenge auf das Haar Wasser zwangsläufig in solchen Mengen auf das Haar gelangt, daß die Trocknungszeiten unakzeptabel lang werden. Aus diesen Problemen heraus resultieren auch starke Schwankungen in der Dosierung der Mittel durch den Verbraucher. Eine weitere Forderung der Verbraucher nach einer ökologischen Alternative zu Haarpflegemitteln mit festigender Wirkung in Form von Schäumen oder Sprays ist auch mit Mitteln auf der Basis überwiegend von Wasser als Lösemittel in noch nicht ausreichendem Maße erfüllt.

Weitere Haarpflegeprodukte zur Frisurengestaltung sind Haarwachse. Haarwachse enthalten als formgebende Komponente in der Regel pflanzliche, tierische oder mineralische Wachse sowie synthetische Polymere und werden als feste Formulierungen, meist in Tiegeln, angeboten. Für die Anwendung wird eine gewisse Menge dem Tiegel entnommen, sodann in der Hand verrieben und auf dem Haar verteilt.

Durch diese Haarwachse wird auf Basis natürlicher Rohstoffe ein guter Halt der Haare bewirkt unter gleichzeitig starker Glanzgebung. Dennoch können die auf dem Markt befindlichen Haarwachse die Wünsche der Anwender hinsichtlich einfacher Applikation und leichter Verteilung auf dem Haar noch nicht vollständig befriedigen. So ist auch hier das genaue, einfache und gleichbleibend reproduzierbare Dosieren ein Problem. Aus den üblichen Tiegeln und Tuben wird häufig entweder zu viel oder zu wenig Produkt entnommen oder insbesondere aus den Tuben führt das Ausdrücken eines Stranges häufig zu Produktverlusten durch eine Verunreinigung der unmittelbaren Umgebung. Ein weiteres nicht zu unterschätzendes Problem ist die mikrobiologische Kontamination durch den Anwender.

Es bestand daher weiterhin die Aufgabe, einfach handhabbare, exakt dosierbare und für jeweils eine Anwendung ausreichend abgepackte Mengen an kosmetischen Mitteln zu entwickeln.

Die festigenden Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger, Haargelen, Haarwachsen oder Haarsprays anzuwenden.

Festigende Haarbehandlungsmittel werden durchaus mehrfach am Tag angewendet. Dabei wird das entsprechende Haarbehandlungsmittel häufig vom Verbraucher stets griffbereit mitgeführt. Dabei ist jedoch das große Volumen der gebräuchlichen Aerosoldosen ein großer Nachteil. Zwar könnten die Aerosoldosen im Volumen reduziert werden, beispielsweise auf Dosen von 100 ml oder 50 ml Inhalt, dann würde der Inhalt jedoch nur für wenige Anwendungen ausreichen. Dies ist für den Verbraucher nicht akzeptabel und erhöht zudem das Abfallaufkommen. Auch die seit einiger Zeit im Handel befindlichen sogenannten Kompakthaarsprays stellen keine befriedigende Lösung dar. In den Formulierungen ist der größte Teil Lösemittel und Treibgas. Beides belastet die Umwelt.

Haarbehandlungsmittel, die dem Haar mehr Volumen und Halt geben, sind bekannt. Die für diese Zwecke üblicherweise eingesetzten kosmetischen Polymere zeigen in wäßrigen, wäßrig-alkoholischen oder alkoholischen Lösungen gute Festigungseigenschaften, die nach der Anwendung mehr oder weniger gut die Haare verformen und festigen und die dem Haar zusätzlich auch mehr Volumen geben können. Häufig hält dieser Effekt aber nicht lange an und schon beim Durchkämmen der Haare geht der erwünschte Volumeneffekt teilweise wieder verloren. Viele der festigenden oder Volumen gebenden Polymere haben häufig unerwünschte Nebeneffekte, die sich dadurch bemerkbar machen, daß das behandelte Haar einen zu rauhen Griff, eine zu hohe Belastung oder eine ungenügende Elastizität aufweist oder sich zuviele sichtbare Rückstände auf dem Haar bilden. Auch eine unzureichende Auswaschbarkeit nach der Anwendung dieser Haarbehandlungsmittel kann ein Problem sein.

Häufig jedoch ist die Ursache für eine Reizung der Haut, Nägel oder der Haare nicht das kosmetische Mittel als solches, sondern vielmehr einzelne Inhaltsstoffe der jeweiligen Zusammensetzungen. Ganz besonders häufig ist dies für die in den kosmetischen Mitteln enthaltenen Konservierungsstoffe zutreffend. Lassen sich während der Herstellung und Verpackung der kosmetischen Mittel noch alle Vorkehrungen seitens des Herstellers der Mittel für eine keimfreie Herstellung treffen, so wird das betreffende kosmetische Mittel im allgemeinen spätestens beim Verbraucher während des sich häufig über Wochen erstreckenden Verwendens des gleichen Gebindes mit Keimen kontaminiert. Seit langem besteht daher seitens der Verbraucher der bislang nahezu unerfüllte Wunsch nach an Konservierungsmitteln freien kosmetischen Zubereitungen.

Die festigenden Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger, Haargelen, Haarwachsen oder Haarsprays anzuwenden.

Haarsprays enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden.

Polymere Verbindungen werden in kosmetischen Mitteln vielfach und mit zunehmender Bedeutung eingesetzt. Sie besitzen zahlreiche Funktionen und Wirkungen, häufig sind sie selbst multifunktional und zeigen in einer einzigen Struktur gleich mehrere erwünschte Wirkungen für das betreffende kosmetische Mittel. So können mit Polymeren kosmetische Mittel gezielt auf die gewünschten rheologischen Eigenschaften eingestellt werden. Beispielsweise können sie Wasser binden und dadurch Viskosität aufbauen. Gleichzeitig bedeutet gebundenes Wasser in kosmetischen Mittel aber auch eine Reduktion der Wasseraktivität, was für die Verkeimung des betreffenden Mittels wichtig sein kann. Wenn die Aktivität des freien Wassers zu gering ist, so können sich darin keine Keime mehr lösen und entwickeln. Das betreffende Mittel muß dann gar nicht oder zumindest deutlich geringer konserviert werden. Multifunktional bedeutet in diesem Zusammenhang, daß der Einsatz eines polymeren Rohstoffes in einer Zusammensetzung mehrere Funktionen gleichzeitig erfüllt.

Es hat viele Bemühungen gegeben, die haarfestigenden Mittel weiterzuentwickeln und zu optimieren. So ersetzten in der Vergangenheit beispielsweise Mittel auf wäßriger Basis Mittel auf der Basis von flüchtigen organischen Verbindungen. Dabei entstand das Problem der geringeren Flüchtigkeit von Wasser im Vergleich zu den Alkoholen, was sich in längeren Trocknungszeiten auf dem Haar niederschlägt. Weiterhin ist aufgrund der häufig schlechteren Löslichkeit polymerer Verbindungen in wäßrigen Systemen diese Umstellung auch häufig mit dem Nachteil verbunden, daß beim Aufbringen der gewünschten Polymermenge auf das Haar Wasser zwangsläufig in solchen Mengen auf das Haar gelangt, daß die Trocknungszeiten unakzeptabel lang werden. Aus diesen Problemen heraus resultieren auch starke Schwankungen in der Dosierung der Mittel durch den Verbraucher. Eine weitere Forderung der Verbraucher nach einer ökologischen Alternative zu Haarpflegemitteln mit festigender Wirkung in Form von Schäumen oder Sprays ist auch mit Mitteln auf der Basis überwiegend von Wasser als Lösemittel in noch nicht ausreichendem Maße erfüllt.

Es gibt nun in jüngster Zeit eine Reihe von Entwicklungen auf dem kosmetischen Gebiet, die einen Bedarf an neuartigen Wirkstoffen oder Wirkstoffkombinationen. neuen Formulierungsformen oder neuen Darreichungsformen geweckt haben. Viele dieser Entwicklungen beruhen dabei nicht ausschließlich auf anwendungstechnischen Nachteilen oder Unzulänglichkeiten der bekannten Mittel, sondern z.B. auf Umweltschutz-Gesichtspunkten, gesetzlichen Auflagen oder anderen "nichttechnischen" Ursachen. Insbesondere der veränderte Lebenswandel der Verbraucher führt dazu, daß der Verbraucher von einem kosmetischen Mittel heute eine einfache, gleichmäßige und gute Dosierung sowie eine jederzeit mögliche schnelle Anwendung der Produkte erwartet.

Festigende Haarbehandlungsmittel werden durchaus mehrfach am Tag angewendet. Dabei wird das entsprechende Haarbehandlungsmittel häufig vom Verbraucher stets griffbereit mitgeführt. Dabei ist jedoch das große Volumen der gebräuchlichen Aerosoldosen ein großer Nachteil. Zwar könnten die Aerosoldosen im Volumen reduziert werden, beispielsweise auf Dosen von 100 ml oder 50 ml Inhalt, dann würde der Inhalt jedoch nur für wenige Anwendungen ausreichen. Dies ist für den Verbraucher nicht akzeptabel und erhöht zudem das Abfallaufkommen. Abhilfe konnten auch hochkonzentrierte, mindestens doppelt bis fünffach so hoch konzentrierte auf den Gehalt an festigenden Polymeren bezogene Haarsprayrezepturen nicht schaffen.

Andererseits haben Produkte in fester Form, welche unmittelbar vor der eigentlichen Anwendung in Wasser gelöst werden, bislang keine Akzeptanz beim Verbraucher gefunden. Dies kann an der unzureichenden Dosiermöglichkeit liegen, weil beispielsweise im Falle von Tabletten nur eine ganze Stylingtablette oder allenfalls eine halbe Tablette in der Hand mit wenig Wasser aufgelöst werden kann. Entsprechende Stylingtabletten werden beispielhaft in der deutschen Patentanmeldung DE 103 12 270 A beschrieben. Abhilfe könnten hier pulverförmige, rieselfähige oder von einem Block abzureibende Zusammensetzungen in einem geeigneten Spendersystem schaffen.

Es besteht daher weiterhin die Aufgabe, entsprechende hochkonzentrierte Mittel zu entwickeln, die hinsichtlich der anwendungstechnischen Eigenschaften, beispielsweise dem langandauernden Halt der Frisur, insbesondere einem langanhaltenden (bis hin zu Tagen) hohen Volumen, der Fülle, der leichten Kämmbarkeit des nassen und trockenen Haares zum Formen der Frisuren, insbesondere im Bereich der Haarspitzen, dem Glanz, dem samtig, geschmeidigen Griff der Haare, der Möglichkeit des Formens von Frisuren und einer kurzen Trocknungszeit bei festigenden Haarpflegemitteln sowie der Flexibilität der geformten Frisur und der Auswaschbarkeit der Zusammensetzungen die vom Verbraucher gesteckten Erwartungen erfüllen. Zusätzlich wird vom Verbraucher eine kleine Verpackung, welche sich bequem transportieren lässt und somit überall verfügbar ist, gewünscht. Ein System, welches öffentlich als Dienstleistung zugänglich wäre, beispielsweise wie Seifenspender in Toiletten, wäre für den Verbraucher ebenfalls vorteilhaft. Trotz der gewünschten kleinen Verpackung soll jedoch der Inhalt für zahlreiche Anwendungen ausreichen und der Zahl der Anwendungen eines handelsüblichen Haarsprays entsprechen oder übertreffen.

Es wurde nun gefunden, dass die Aufgabe gelöst wird durch ein Kit, umfassend

a) festigendes Haarbehandlungsmittel in fester Form, welches aufweist:

- mindestens 1,0 Gew.% mindestens eines festigenden Polymers,
- mindestens 0,1 Gew.% eines Auflösungsbeschleunigers und welches in Form eines Granulats oder als fester Block vorliegt,

b) Spendersystem, mit Hilfe dessen das Haarbehandlungsmittel angewendet wird und das aus einer Mühle oder Reibe besteht, mit deren Hilfe von dem festen Block oder von dem Granulat ein Pulver abgerieben wird.

Selbstverständlich umfasst die vorliegende Erfindung auch mehr oder weniger kugelförmige Formkörper von mehreren Millimeter Durchmesser, welche erst unmittelbar vor der Anwendung zu einem Pulver gemahlen oder gerieben werden. Alle derartigen Zusammensetzungen werden dabei als feinteiliges Pulver entweder in der Hand oder nach dem Aufbringen des Pulvers auf dem Haar mit wenig Wasser befeuchtet. Im Anschluß daran wird mit diesen Zubereitungen die Frisur wie üblich gestylt, aufgebaut und/oder gefestigt.

Ein weiterer Gegenstand der vorliegenden Anmeldung ist ein Verfahren zur Behandlung von Haar, bei dem ein festigendes Haarbehandlungsmittel in fester Form, welches aufweist:

- mindestens 1,0 Gew.% mindestens eines festigenden Polymers,
- mindestens 0,1 Gew.% eines Auflösungsbeschleunigers und welches in Form eines Granulats oder als fester Block vorliegt, mit Hilfe eines Spendersystems, das aus einer Mühle oder Reibe besteht mit deren Hilfe von dem festen Block oder von dem Granulat ein Pulver abgerieben wird, auf das Haar aufgetragen wird.

Das Verfahren hat gegenüber dem Stand der Technik den großen Vorteil, daß es nur eine Zusammensetzung für alle verschiedenen Festigungsgrade gibt. Durch die Menge wird geregelt, ob die Festigung gering oder bis hin zu einer starken Festigung reichen soll. Ein weiterer Vorteil ist, dass gegebenenfalls keinerlei festigende Produkte seitens der Verbraucher mehr mitgeführt werden müssen.

Derartige Konzepte sind bislang weder im Handel erhältlich, noch in der Literatur beschrieben. Sie sind beispielsweise in einer der folgenden Ausführungsformen umsetzbar:

Ausführungsformen des Spendersystemes:

In einer ersten erfindungsgemäßen Ausführungsform umfasst das Spendersystem einen mit einem Mahlwerk versehenen Behälter. Die Zusammensetzungen werden in Blockform in den Vorratsteil des Spenderbehälters gegeben. Die Abmessungen in den drei räumlichen Dimensionen bzw. im Durchmesser bei elliptischen oder kugelförmigen Querschnitten des Aufnahmebehälters für den Block mit der erfindungsgemäßen Zusammensetzung betragen von 0,5 cm bis zu 25 cm. Das gesamte System kann beispielsweise in Toiletten montiert werden. Wie bei den bekannten Seifenspendern, welche mit festen Stückseifen bestückt sind, von welchen mittels einer Reibe vor dem Gebrauch eine bestimmte Menge abgeraspelt wird, so können auch diese festigenden festen Zusammensetzungen in derartigen Reiben bevorratet werden, um jederzeit dem Verbraucher zu jeder Gelegenheit zur Verfügung zu stehen. Der feste Block der erfindungsgemäßen Zusammensetzung kann dabei alle Formen und Geometrien einnehmen. Beschränkt wird dies durch die Formgebung des Vorratsbehältnisses oberhalb des Mahlwerkes.

Desweiteren kann durch die Wahl der Art des Mahlwerkes die Darreichungsform variiert werden. Ähnlich Küchengerätschaften dient ein Mahlwerk mit länglichen Schlitzen zur Erzeugen von einem Streifenförmigen Produkte. Bei der Wahl von unterschiedlichen, Raspelartigen Mahlwerken kann Produktpulver in unterschiedlichen Feinheitsgraden abgegeben werden. Im Rahmen der Erfindung ist es denkbar dem Endnutzer des Spendersystems unterschiedliche Mahlwerke an die Hand zu geben, so dass er je nach Situation und gewünschter Stylingart die Feinheit des Produktpulvers variabel einstellen kann. Diese Einstellbarkeit kann entweder durch die Auswechselbarkeit des Mahlwerks oder durch Verstellbarkeit der einzelnen Mahlelemente zueinander erzeugt werden.

Bei Großverbrauchen, wie beispielsweise Friseurläden ist auch eine elektrische Betätigung des Mahlwerks denkbar. In diesem Fall können schnell und ohne Zeitverlust die jeweils benötigte Menge an Produkt frisch abgegeben werden. Diese Spendersysteme können sowohl stationär, beispielsweise an der Wand befestigt, oder aber mobil, wie elektrische Pfefferstreuer ausgeführt sein.

In einer erfindungsgemäßen Ausgestaltungsform dieser Ausführungsform wird eine Zusammensetzung zum Frisieren und/oder Stylen und/oder Festigen von Haaren als fester Stückkörper konfektioniert. Von diesem festen Stückkörper kann dann die benötigte und gewünschte Menge der Zusammensetzung mit einer Reibe abgerieben werden. Hierbei ist nicht zwingend ein den Stückkörper umgebender Behälter notwendig. Vielmehr kann der Stückkörper auch nur in Papier oder Folie verpackt sein. Als Reibe kann eine Handreibe verwendet werden. Erfindungsgemäß sind alle Arten von Reiben verwendbar. Hierzu kann die Reibe beispielsweise flach oder gewölbt ausgebildet sein oder in der Art einer Muskatreibe. Die geometrische Form des Stückkörpers ist hierbei beliebig. Der erfindungsgemäße Stückkörper kann in Form einer Kugel, eines Quaders, eines Tetraeders, eines Würfels etc. vorliegen.

Vorzugsweise ist die den Stückkörper umgebende Verpackung aus einem Material, welches mitgerieben werden kann und sich nicht negativ auf die gewünschten Eigenschaften des Produktes auswirkt. Insbesondere flüssigkeitslösliche Verpackungen sind möglich. Um sicherzustellen, dass diese Verpackung sich nicht bereits bei Lagerung oder Transport ungewollt auflöst, ist es sinnvoll die Auflöseeigenschaft derart zu gestalten, dass die Auflösung nur bei großem Oberflächen/Volumenverhältnis, also im geriebenen Zustand möglich wird. Es ist also vorteilhaft die Auflöseeigenschaft mit einer hohen volumenbezogenen Oberfläche zu koppeln.

In einer zweiten Ausführungsform wird eine Zusammensetzung zum Frisieren und/oder Festigen und/oder Stylen von Haar bereits als fertiges, fließ- und rieselfähiges Pulver konfektioniert. Als Spendersystem für eine derartige Zusammensetzung kann im Prinzip jedes handelsübliche Produkt verwendet werden, aus welchem Feststoffe als Pulver ausgestreut werden können. Hier können beispielsweise Salz und Pfefferstreuer, Streuer für Puderzucker etc. Verwendung finden. Wesentlich ist hierbei jedoch, dass die Größe und die Form der Öffnungen im Streukopf auf die Größe und die Form des Pulvers oder Granulates abgestimmt ist. Selbstverständlich können auch ähnlich wie bei bekannten Pfeffer- oder Gewürzstreuern im Verschlußteil für das Gefäß mehrere siebartige auf die Größe und die Form des Pulvers oder Granulates abgestimmte Lochplatten segmentartig untergebracht sein. Die Bohrungen der Siebplatte können dann unterschiedlich sein, so dass bei einem Schüttvorgang unterschiedliche Mengen an Pulver oder Granulat entnommen werden können.

Vorteilhaft bei einer derartigen Ausführungsform ist weiterhin das vorsehen eine mechanischen oder sonstige Einrichtung zur Zerkleinerung und/oder Zerstörung von unerwünschten Agglomeraten im pulverförmigen oder granulierten Produkt. Eine mögliche Ausgestaltung ist das Vorsehen von mindestens einem abgeflachten Element, welches auf der Innenseite der Öffnungen an diesen entlang bewegt werden kann. Eine einfache Ausgestaltung ist ein rotierendes Element mit zwei oder mehr Schabelementen, welche an den Öffnungen anstehende Agglomerate zerstören oder soweit zerkleinern, dass diese durch die Öffnung passen. Diese Einrichtungen sind dann hilfreich, wenn das Produkt längere gelagert wird, insbesondere, wenn es sich um ein hygroskopisches Produkt handelt.

In einer dritten erfindungsgemäßen Form wird eine Zusammensetzung zum Frisieren und/oder Festigen und/oder Stylen von Haar als fester Formkörper mit einer Größe von wenigen Millimetern Durchmesser konfektioniert. Diese Formkörper können dann in einem Spendersystem wie beispielsweise eine handelsübliche Mühle, wie sie auch für Gewürze oder Salz angeboten wird, frisch gemahlen portionsweise entnommen werden. Das Mahlen der Formkörper kann dabei selbstverständlich mechanisch von Hand oder mit Hilfe einer schnurgebundenen oder schnurlosen, Batterie betriebenen Mahlwerkes erfolgen.

In allen bisherigen Ausführungsformen kann das feinteilige Pulver, nachdem es auf das Haar aufgebracht wurde, mit Hilfe eines Pinsels, eines Kammes oder einer Bürste noch besser auf dem Haar verteilt werden. Auch kann das Wasser mit Hilfe eines angefeuchteten Pinsels auf das Pulver und das Haar aufgebracht werden. Diese Vorgehensweise erlaubt es einerseits sich nicht die Hände zu beschmutzen, andererseits eine gezielte und gleichmäßige Auftragung auf dem Haar zu ermöglichen. Als Pinsel ist im Prinzip jeglicher Pinsel, wie er üblicherweise im Zusammenhang mit kosmetischen Anwendungen auf der Haut oder dem Haar verwendet wird, geeignet. Ganz besonders geeignet ist ein Glitterpinsel. Derartige Glitterpinsel haben einen dichten Borstenstand und einen größeren, meist runden oder ovalen Durchmesser von mehreren Millimeter bis hin zu 5 Zentimetern. Aber auch jede andere äußere Form des Pinsels, wie Quader, Würfel, Pyramide, Tetraeder etc. ist erfindungsgemäß geeignet.

Selbstverständlich ist erfindungsgemäß auch die Verwendung einer entsprechenden Bürste, eines Pinsels beispielsweise wie für Mascara, geeignet. Die Borsten des Pinsels können aus synthetischem Material oder auch aus natürlichem Material sein. Bevorzugt ist natürliches Material. Naturborsten können aus Pferdehaar, Kamelhaar, Schafwolle usw. sein. Bevorzugt ist die Verwendung von Pferdehaar.

In allen Ausführungsformen kann die feinteilige Zusammensetzung prinzipiell auf dem trockenen oder dem leicht feuchten bis nassen Haar angewendet werden. Bevorzugt ist die Anwendung auf dem leicht feuchten bis nassen Haar. In den Fällen, in welchen mit einem Pinsel gearbeitet wird, kann auch der Pinsel bereits feucht sein, um größere Mengen auf dem Haar anzuwenden. Selbstverständlich kann der Pinsel aber auch trocken und nur das Haar feucht bis nass sein.

Das erfindungsgemäße Pulver, Granulat bzw. der Blockkörper

Im folgenden Text wird überwiegend der Begriff "Formkörper" verwendet. Unter Formkörper im Sinne der vorliegenden Erfindung ist sowohl das Pulver als auch das Granulat zu verstehen. Unter dem Begriff Blockkörper ist ein festes Stück zu verstehen. Der Begriff "feste Stücke" wird daher auch im folgenden verwendet.

Weiterhin können die Granulate nach üblichen Tablettierungsverfahren hergestellt werden. Der Begriff "Tablette" und dessen Abwandlungen stehen daher gleichbedeutend im Sinne der Erfindung mit den erfindungsgemäßen Granulaten.

Eine Ausgestaltungsform besteht darin, die erfindungsgemäßen Zusammensetzungen als Blockkörper zu formulieren. Im folgenden wird daher diese Ausgestaltung detailliert beschrieben. Die geometrischen räumlichen Abmessungen dieser Ausführungsform sind an die Geometrie der Vorratsbehälter anzupassen. Die Abmessungen dieser Vorratsbehälter sind bereits bei der Beschreibung dieser Ausführungsform beschrieben. Vorzugsweise haben diese Blockkörper Dimensionen und Abmessungen wie beispielsweise Syndet- oder Seifenstücke.

Die erfindungsgemäßen Formkörper können jedwede geometrische Form annehmen, wie beispielsweise konkave, konvexe, bikonkave, bikonvexe, kubische, tetragonale, orthorhombische, zylindrische, sphärische, zylindersegmentartige, scheibenförmige, tetrahedrale, dodecahedrale, octahedrale, konische, pyramidale, ellipsoide, fünf-, sieben- und achteckig-prismatische sowie rhomboedrische Formen. Auch völlig irreguläre Grundflächen wie Pfeil- oder Tierformen, Bäume, Wolken usw. können realisiert werden. Die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt und Formkörper mit sphärischer Geometrie sind erfindungsgemäß bevorzugt. Besonders bevorzugt sind Formkörper in Gestalt sphärischer Geometrie.

Die zylinderförmige Ausgestaltung erfaßt dabei die Darbietungsform von der Tablette bis zu kompakten Zylinderstücken mit einem Verhältnis von Höhe zu Durchmesser größer 1. Weist der Basisformkörper Ecken und Kanten auf, so sind diese vorzugsweise abgerundet. Als zusätzliche optische Differenzierung ist eine Ausführungsform mit abgerundeten Ecken und abgeschrägten ("angefasten") Kanten bevorzugt.

In einer bevorzugten Ausführungsform können die portionierten Preßlinge dabei jeweils als voneinander getrennte Einzelelemente ausgebildet sein, die der vorbestimmten Dosiermenge des kosmetischen Wirkstoffs entspricht. Ebenso ist es aber möglich, Preßlinge auszubilden, die eine Mehrzahl solcher Masseneinheiten in einem Preßling verbinden, wobei insbesondere durch vorgegebene Sollbruchstellen die leichte Abtrennbarkeit portionierter kleinerer Einheiten vorgesehen ist. Die Ausbildung der portionierten Preßlinge als Tabletten in Zylinder- oder Quaderform kann zweckmäßig sein, wobei ein Durchmesser/Höhe-Verhältnis im Bereich von etwa 0,1 : 10 bis 10 : 0,1 bevorzugt ist. Handelsübliche Hydraulikpressen, Exzenterpressen oder Rundläuferpressen sind geeignete Vorrichtungen insbesondere zur Herstellung derartiger Preßlinge.

Die bevorzugte Raumform der erfindungsgemäßen Formkörper weist eine rechteckige Grundfläche auf, wobei die Höhe der Formkörper kleiner ist als die kleinere Rechteckseite der Grundfläche. Abgerundete Ecken sind bei dieser Angebotsform bevorzugt.

Ein weiterer bevorzugter Formkörper, der hergestellt werden kann, hat eine platten- oder tafelartige Struktur mit abwechselnd dicken langen und dünnen kurzen Segmenten, so daß einzelne Segmente von diesem "Riegel" an den Sollbruchstellen, die die kurzen dünnen Segmente darstellen, abgebrochen und derartig portioniert zum Einsatz kommen können. Dieses Prinzip des "riegelförmigen" Formkörpers kann auch in anderen geometrischen Formen, beispielsweise senkrecht stehenden Dreiecken, die lediglich an einer ihrer Seiten längsseits miteinander verbunden sind, verwirklicht werden.

Enthalten die erfindungsgemäßen Formkörper mindestens zwei kosmetische Wirkstoffe, kann es in einer weiteren Ausführungsform vorteilhaft sein, die verschiedenen Komponenten nicht ausschließlich zu einer einheitlichen Tablette zu verpressen. Bei der Tablettierung werden in dieser Ausführungsform Formkörper erhalten, die mehrere Schichten, also mindestens zwei Schichten, aufweisen. Dabei ist es auch möglich, daß diese verschiedenen Schichten unterschiedliche Lösegeschwindigkeiten aufweisen. Hieraus können vorteilhafte anwendungstechnische Eigenschaften der Formkörper resultieren. Falls beispielsweise Komponenten in den Formkörpern enthalten sind, die sich wechselseitig negativ beeinflussen, so ist es möglich, die eine Komponente in der schneller löslichen Schicht zu integrieren und die andere Komponente in eine langsamer lösliche Schicht einzuarbeiten, so daß die Komponenten nicht bereits während des Lösevorgangs miteinander reagieren.

Der Schichtaufbau der Formkörper kann dabei sowohl stapelartig erfolgen, wobei ein Lösungsvorgang der inneren Schicht(en) an den Kanten des Formkörpers bereits dann erfolgt, wenn die äußeren Schichten noch nicht vollständig gelöst sind. Bei der stapelförmigen Anordnung kann die Stapelachse beliebig zur Tablettenachse angeordnet sein. Die Stapelachse kann also beispielsweise bei einer zylinderförmigen Tablette parallel oder senkrecht zur Höhe des Zylinders liegen.

Es kann aber auch gemäß einer weiteren Ausführungsform bevorzugt sein, wenn eine vollständige Umhüllung der inneren Schicht(en) durch die jeweils weiter außen liegende(n) Schicht(en) erreicht wird, was zu einer Verhinderung der frühzeitigen Lösung von Bestandteilen der inneren Schicht(en) führt. Bevorzugt sind Formkörper, bei denen die Schichten mit den verschiedenen Wirkstoffen sich umhüllen. Beispielsweise sei eine Schicht (A) vollständig von der Schicht (B) und diese wiederum vollständig von der Schicht (C) umhüllt. Ebenso können Formkörper bevorzugt sein, bei denen z.B. die Schicht (C) vollständig von der Schicht (B) und diese wiederum vollständig von der Schicht (A) umhüllt sind.

Ähnliche Effekte lassen sich auch durch Beschichtung ("coating") einzelner Bestandteile der zu verpressenden Zusammensetzung oder des gesamten Formkörpers erreichen. Hierzu können die zu beschichtenden Körper beispielsweise mit wäßrigen Lösungen oder Emulsionen bedüst werden, oder aber über das Verfahren der Schmelzbeschichtung einen Überzug erhalten.

Die erfindungsgemäß hergestellten (Mulden)-Formkörper können ganz oder teilweise mit einer Beschichtung versehen werden. Verfahren, in denen eine Nachbehandlung im Aufbringen einer Coatingschicht auf die Formkörperfläche(n), in der/denen sich die befüllte(n) Mulde(n) befinden, oder im Aufbringen einer Coatingschicht auf den gesamten Formkörper besteht, sind erfindungsgemäß bevorzugt.

Es kann erfindungsgemäß bevorzugt sein, einzelne Wirkstoffe vor ihrer Einarbeitung in den Formkörper separat zu verkapseln; so ist es beispielsweise denkbar, besonders reaktive Komponenten oder auch die Duftstoffe in verkapselter Form einzusetzen.

Die Herstellung der erfindungsgemäßen Formkörper erfolgt zunächst durch das trockene Vermischen der Bestandteile, die ganz oder teilweise vorgranuliert sein können, und anschließendes Informbringen, insbesondere Verpressen zu Tabletten, wobei auf bekannte Verfahren zurückgegriffen werden kann. Zur Herstellung der erfindungsgemäßen Formkörper wird das Vorgemisch in einer sogenannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen.

Die erfindungsgemäßen Formkörper können nach der Herstellung verpackt werden, wobei sich der Einsatz bestimmter Verpackungssysteme besonders bewährt hat, da diese Verpackungssysteme einerseits die Lagerstabilität der Inhaltsstoffe erhöhen, andererseits gegebenenfalls aber auch die Langzeithaftung der Muldenfüllung deutlich verbessern. Der Begriff "Verpackungssystem" kennzeichnet dabei im Rahmen der vorliegenden Erfindung immer die Primärverpackung der Formkörper, d.h. die Verpackung, die an ihrer Innenseite direkt mit der Formkörperoberfläche in Kontakt ist. An eine optionale Sekundärverpackung werden keinerlei Anforderungen gestellt, so daß hier alle üblichen Materialien und Systeme eingesetzt werden können.

Erfindungsgemäß bevorzugt sind Verpackungssysteme, die nur eine geringe Feuchtigkeitsdurchlässigkeit aufweisen. Auf diese Weise läßt sich der erfindungsgemäße Formkörper über einen längeren Zeitraum erhalten, auch wenn beispielsweise hygroskopische Komponenten in den Formkörpern eingesetzt werden. Besonders bevorzugt sind Verpackungssysteme, die eine Feuchtigkeits-dampfdurchlässigkeitsrate von $0,1 \text{ g/m}^2$/Tag bis weniger als $20 \text{ g/m}^2$/Tag aufweist, wenn das Verpackungssystem bei 23°C und einer relativen Gleichgewichtsfeuchtigkeit von 85% gelagert wird. Die genannten Temperatur- und Feuchtigkeitsbedingungen sind die Prüfbedingungen, die in der DIN-Norm 53122 genannt werden, wobei laut DIN 53122 minimale Abweichungen zulässig sind ($23 \pm 1$°C, $85 \pm 2$% rel. Feuchte). Die Feuchtigkeitsdampfdurchlässigkeitsrate eines gegebenen Verpackungssystems bzw. Materials läßt sich nach weiteren Standardmethoden bestimmen und ist beispielsweise auch im ASTM-Standard E-96-53T ("Test for measuring Water Vapor transmission of Materials in Sheet form") und im TAPPI Standard T464 m-45 ("Water Vapor Permeability of Sheet Materials at high temperature an Humidity") beschrieben. Das Meßprinzip gängiger Verfahren beruht dabei auf der Wasseraufnahme von wasserfreiem Calciumchlorid, welches in einem Behälter in der entsprechenden Atmosphäre gelagert wird, wobei der Behälter an der Oberseite mit dem zu testenden Material verschlossen ist. Aus der Oberfläche des Behälters, die mit dem zu testenden Material verschlossen ist (Permeationsfläche), der Gewichtszunahme des Calciumchlorids und der Expositionszeit läßt sich die Feuchtigkeitsdampfdurchlässigkeitsrate nach

$$FDDR = \frac{24 \cdot 10000}{A} \cdot \frac{x}{y} \left[ g/m^2/24h \right]$$

berechnen, wobei A die Fläche des zu testenden Materials in cm$^2$, x die Gewichtszunahme des Calciumchlorids in g und y die Expositionszeit in h bedeutet.

Die relative Gleichgewichtsfeuchtigkeit, oft als "relative Luftfeuchtigkeit" bezeichnet, beträgt bei der Messung der Feuchtigkeitsdampfdurchlässigkeitsrate im Rahmen der vorliegenden Erfindung 85% bei 23°C. Die Aufnahmefähigkeit von Luft für Wasserdampf steigt mit der Temperatur bis zu einem jeweiligen Höchstgehalt, dem sogenannten Sättigungsgehalt, an und wird in g/m$^3$ angegeben. So ist beispielsweise 1 m$^3$ Luft von 17° mit 14,4 g Wasserdampf gesättigt, bei einer Temperatur von 11° liegt eine Sättigung schon mit 10 g Wasserdampf vor. Die relative Luftfeuchtigkeit ist das in Prozent ausgedrückte Verhältnis des tatsächlich vorhandenen Wasserdampf-Gehalts zu dem der herrschenden Temperatur entsprechenden Sättigungs-Gehalt. Enthält beispielsweise Luft von 17° 12 g/m$^3$ Wasserdampf, dann ist die relative Luftfeuchtigkeit = (12/14,4) 100 = 83%. Kühlt man diese Luft ab, dann wird die Sättigung (100% r. L.) beim sogenannten Taupunkt (im Beispiel: 14°) erreicht, d.h., bei weiterem Abkühlen bildet sich ein Niederschlag in Form von Nebel (Tau). Zur quantitativen Bestimmung der Feuchtigkeit benutzt man Hygrometer und Psychrometer.

Die relative Gleichgewichtsfeuchtigkeit von 85% bei 23°C läßt sich beispielsweise in Laborkammern mit Feuchtigkeitskontrolle je nach Gerätetyp auf +/- 2% r.L. genau einstellen. Auch über gesättigten Lösungen bestimmter Salze bilden sich in geschlossenen Systemen bei gegebener Temperatur konstante und wohldefinierte relative Luftfeuchtigkeiten aus, die auf dem Phasen-Gleichgewicht zwischen Partialdruck des Wassers, gesättigter Lösung und Bodenkörper beruhen.

Die Kombinationen aus Formkörper und Verpackungssystem können selbstverständlich ihrerseits in Sekundärverpackungen, beispielsweise Kartonagen oder Trays, verpackt werden, wobei an die Sekundärverpackung keine weiteren Anforderungen gestellt werden müssen. Die Sekundärverpackung ist demnach möglich, aber nicht notwendig.

Das Verpackungssystem der erfindungsgemäßen Kombination kann aus den unterschiedlichsten Materialien bestehen und beliebige äußere Formen annehmen. Aus ökonomischen Gründen und aus Gründen der leichteren Verarbeitbarkeit sind allerdings Verpackungssysteme bevorzugt, bei denen das Verpackungsmaterial ein geringes Gewicht hat, leicht zu verarbeiten und kostengünstig sowie ökologisch verträglich ist.

Die Zusammensetzung

Die erfindungsgemäße Zusammensetzung kann um eine rasche und vollständige Auflösung mit Wasser zu erzielen, mindestens einen Auflösungsbeschleuniger enthalten. Der Begriff Auflösungsbeschleuniger umfaßt dabei Gasentwickelnde Komponenten, vorgebildete und eingeschlossene Gase, Sprengmittel sowie deren Mischungen.

Unter dem Begriff Auflösungsbeschleuniger, Formkörpersprengmittel, Spreng- oder Desintegrationsmittel sind Stoffe zu verstehen, die Tabletten zugegeben werden, um deren Zerfall beim Inkontaktbringen mit Wasser oder anderen Lösemitteln zu beschleunigen. Übersichten hierzu finden sich z.B. in J.Pharm.Sci. 61 (1972), Römpp Chemielexikon, 9. Auflage, Band 6, S. 4440 sowie und Voigt "Lehrbuch der pharmazeutischen Technologie" (6. Auflage, 1987, S. 182-184). Diese Stoffe vergrößern bei Zutritt des Lösemittels, beispielsweise Wasser, ihr Volumen, wobei einerseits das Eigenvolumen vergrößert (Quellung), andererseits auch über die Freisetzung von Gasen ein Druck erzeugt werden kann, der die Tablette in kleinere Partikel zerfallen läßt. In der Pharmazie werden zu diesem Zweck Celluloseabkömmlinge oder Polymere eingesetzt.

In einer Ausführungsform der vorliegenden Erfindung werden als Auflösungsbeschleuniger Gasentwickelnde Komponenten eingesetzt. Diese Komponenten reagieren bei Kontakt mit Wasser miteinander unter insitu Bildung von Gasen, die in der Tablette einen Druck erzeugen, der die Tablette in kleinere Partikel zerfallen läßt. Ein Beispiel für ein derartiges System sind spezielle Kombinationen von geeigneten Säuren mit Basen. Bevorzugt sind ein-, zwei- oder dreiwertige Säuren mit einem $pK_a$-Wert von 1,0 bis 6,9. Bevorzugte Säuren sind Citronensäure, Äpfelsäure, Maleinsäure, Malonsäure, Itaconsäure, Weinsäure, Oxalsäure, Glutarsäure, Glutaminsäure, Milchsäure, Fumarsäure, Glykolsäure sowie deren Mischungen. Besonders bevorzugt ist Citronensäure. Ganz besonders bevorzugt kann es sein, die Citronensäure in Teilchenform einzusetzen, wobei die Teilchen einen Durchmesser unterhalb von 1000 μm, insbesondere kleiner als 700 μm, ganz besonders bevorzugt kleiner als 400 μm, aufweisen. Weitere alternative geeignete Säuren sind die Homopolymere oder Copolymere von Acrylsäure, Maleinsäure, Methacrylsäure oder Itaconsäure mit einem Molekulargewicht von 2000 bis 200 000. Besonders bevorzugt sind Homopolymere der Acrylsäure und Copolymere aus Acrylsäure und Maleinsäure. Bevorzugte Basen sind erfindungsgemäß Alkalimetallsilikate, Carbonate, Hydrogencarbonate sowie deren Mischungen. Metasilicate, Hydrogencarbonate und Carbonate sind besonders bevorzugt, Hydrogencarbonate sind ganz besonders bevorzugt. Besonders bevorzugt sind teilchenförmige Hydrogencarbonate mit einem Teilchendurchmesser von weniger als 1000 μm, insbesondere weniger als 700 μm, ganz besonders bevorzugt weniger als 400 μm. Natrium oder Kaliumsalze der oben genannten Basen sind besonders bevorzugt. Diese Gasentwickelnden Komponenten sind in den erfindungsgemäßen Färbeformkörpern bevorzugt in einer Menge von mindestens 10 Gew.-%, insbesondere von mindestens 20 Gew.-%, enthalten.

In einer weiteren Ausführungsform der vorliegenden Erfindung ist das Gas vorgebildet oder eingeschlossen, so daß bei Einsetzen der Auflösung des Formkörpers die Gasentwicklung beginnt und die weitere Auflösung beschleunigt. Beispiele geeigneter Gase sind Luft, Kohlendioxid, $N_2O$, Sauerstoff und/oder weitere nicht-toxische, nichtbrennbare Gase.

In einer weiteren, besonders bevorzugten Ausführungsform der vorliegenden Erfindung werden als Auflösungsbeschleuniger Desintegrationshilfsmittel, sogenannte Formkörpersprengmittel, in die als Formkörper vorliegende erfindungsgemäße Zusammensetzung eingearbeitet, um die Zerfallszeiten zu verkürzen.

Diese Stoffe, die auch aufgrund ihrer Wirkung als "Spreng"mittel bezeichnet werden, vergrößern bei Wasserzutritt ihr Volumen (Quellung). Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden im Rahmen der vorliegenden Erfindung Desintegrationsmittel auf Cellulosebasis eingesetzt, so daß bevorzugte Formkörper ein solches Desintegrationsmittel auf Cellulosebasis in Mengen von 0,5 bis 50 Gew.-%, vorzugsweise 3 bis 30 Gew.-%, bezogen auf den gesamten Formkörper enthalten. Reine Cellulose weist die formale Bruttozusammensetzung $(C_6H_{10}O_5)_n$ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die HydroxyGruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose kann nicht in feinteiliger Form eingesetzt, sondern vor dem Zumischen zu den zu verpressenden Vorgemischen in eine gröbere Form überführt, beispielsweise granuliert oder kompaktiert. Die Teilchengrößen solcher Desintegrationsmittel liegen zumeist oberhalb 200 μm, vorzugsweise zu mindestens 90 Gew.-% zwischen 300 und 1600 μm und insbesondere zu mindestens 90 Gew.-% zwischen 400 und 1200 μm. Die erfindungsgemäßen Desintegrationshilfsmittel sind beispielsweise im Handel unter der Bezeichnung Arbocel® von der Firma Rettenmaier erhältlich. Ein bevorzugtes Desintegrationshilfsmittel ist beispielsweise Arbocel® TF-30-HG.

Als Desintegrationsmittel auf Cellulosebasis oder als Bestandteil dieser Komponente wird bevorzugt mikrokristalline Cellulose verwendet. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 μm aufweisen und beispielsweise zu Granulaten mit einer mittleren Teilchengröße von 200 μm kompaktierbar sind. Geeignete mikrokristalline Cellulose ist beispielsweise unter dem Handelsnamen Avicel® kommerziell erhältlich.

Weitere Sprengmittel, die im Sinne der Erfindung zugegen sein können, wie z.B. Kollidon, Alginsäure und deren Alkalisalze, amorphe oder auch teilweise kristalline Schichtsilicate (Bentonite), Polyacrylate, Polyethylenglycole sind beispielsweise den Druckschriften WO 98/40462 (Rettenmaier), WO 98/55583 und WO 98/55590 (Unilever) und WO 98/40463, DE 19709991 und DE 19710254 (Henkel) zu entnehmen. Auf die Lehre dieser Schriften wird ausdrücklich Bezug genommen. Die nach dem erfindungsgemäßen Verfahren erhältlichen Sprengmittel können im Formkörper makroskopisch betrachtet homogen verteilt vorliegen, mikroskopisch gesehen bilden sie jedoch herstellungsbedingt Zonen erhöhter Konzentration.

Die beschleunigte Auflösung der als Formkörper vorliegenden erfindungsgemäßen kosmetischen Zusammensetzungen kann erfindungsgemäß auch durch Vorgranulierung der weiteren Bestandteile des Formkörpers erreicht werden.

In einer bevorzugten Ausführungsform der als Formkörper vorliegenden erfindungsgemäßen kosmetischen Zusammensetzungen enthalten diese zusätzlich zum Auflösungsbeschleuniger ein Gemisch aus Stärke und mindestens einem Saccharid. Die Verwendung von Disacchariden gemäß dieser Ausführungsform ist bevorzugt. Das besagte Gemisch liegt bevorzugt in einem Gewichtsverhältnis von Stärke und den eingesetzten Sacchariden von 10 : 1 bis 1 : 10, besonders bevorzugt von 1 : 1 bis 1 : 10, ganz besonders bevorzugt von 1 : 4 bis 1 : 7 in dem Formkörper vor.

Die verwendeten Disaccharide sind bevorzugt ausgewählt aus Lactose, Maltose, Saccharose, Trehalose, Turanose, Gentiobiose, Melibiose und Cellobiose. Besonders bevorzugt werden Lactose, Maltose und Saccharose und ganz besonders bevorzugt Lactose in den erfindungsgemäßen Formkörpern eingesetzt.

Die Stärke-Disaccharid-Mischung ist in dem Formkörper in einer Menge von 5 bis 60 Gew.%, bevorzugt von 20 bis 40 Gew.% bezogen auf die Masse des gesamten Formkörpers, enthalten.

Polymere, welche das Haar fixieren, die sogenannten festigenden Polymere, tragen zum Halt und/oder zum Aufbau des Haarvolumens, der Haarfülle der Gesamtfrisur bei. Filmbildende Polymere und Gumme sind daher generell typische Substanzen für Haarbehandlungsmittel wie Haarfestiger, Haarschäume, Haarwachse, Haarsprays. Als solche finden sie bevorzugt Verwendung in den erfindungsgemäßen Pulvern oder Formkörpern. Substanzen, welchen dem Haar weiterhin hydrophobe Eigenschaften verleihen, sind hierbei bevorzugt, weil sie die Tendenz des Haares Feuchtigkeit, also Wasser zu absorbieren, verringern. Dadurch wird das schlaffe Herunterhängen der Haarsträhnen vermindert und somit wird ein langanhaltender Frisurenaufbau und -erhalt gewährleistet. Als Testmethode hierfür wird häufig der sogenannte curl-retention - Test angewendet. Die Verwendung mindestens eines dieser Polymere in den erfindungsgemäßen Mitteln ist daher erfindungsgemäß bevorzugt. Ganz besonders bevorzugt sind aus dieser Gruppe der Polymere diejenigen, welche zusätzlich auch festigende Eigenschaften aufweisen. Es ist jedoch auch erfindungsgemäß bevorzugt, wenn in den erfindungsgemäßen Mitteln mindestens ein festigendes und ein filmbildendes Polymer verwendet werden. Höchst bevorzugt ist es, wenn beide Polymere gleichzeitig, wenn auch gegebenenfalls in unterschiedlicher Ausprägung sowohl festigende als auch filmbildende Eigenschaften aufweisen.

Festigende Polymere tragen zum Halt und/oder zum Aufbau des Haarvolumens, der Haarfülle der Gesamtfrisur bei. Diese sogenannten festigenden Polymere sind gleichzeitig auch filmbildende Polymere und daher generell typische Substanzen für Haarbehandlungsmittel wie Haarfestiger, Haarschäume, Haarwachse, Haarsprays. Die Filmbildung kann dabei durchaus punktuell sein und nur einige Fasern miteinander verbinden.

Wegen der Bedeutung gerade der festigenden Polymere sollen diese daher explizit in Form ihrer INCI - Namen aufgelistet werden. In dieser Liste der erfindungsgemäß ganz besonders bevorzugt zu verwendenden Polymere finden sich somit selbstverständlich gerade auch die kationischen Polymere wieder.

Beispiele für gebräuchliche filmbildende, festigende Polymere sind: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Ganz besonders bevorzugt sind Acrylates/t-Butylacrylamide Copolymer, Octylacrylamide/Acrylates/Butylaminoethyl Methacrylate Copolymer, Polyurethane-1, Polyvinylcaprolactam und VP/VA Copolymer.

Das filmbildende und/oder festigende Polymer (A) ist in dem erfindungsgemäßen Mittel vorzugsweise in einer Menge von 3,0 bis 40 Gew.%, besonders bevorzugt von 3,0 bis 30 Gewichtsprozent, ganz besonders bevorzugt in einer Menge von 3,0 bis 20 Gewichtsprozent enthalten. Selbstverständlich können auch mehrere filmbildende und/oder festigende Polymere in dem erfindungsgemäßen Mittel enthalten sein. Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Auf dem Haar verbleibende Zubereitungen haben sich als wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d.h. in der Regel mehr als 12 Stunden, auf dem Haar.

Gemäß weiteren bevorzugten Ausführungsformen kann es sich bei den erfindungsgemäßen Mitteln aber beispielsweise um festigende Mittel wie Haarfestiger, Schaumfestiger, Styling Gels und Fönwellen, handeln.

Weitere Gegenstände der vorliegenden Erfindung sind die Verwendung einer erfindungsgemäßen Zubereitung zur Restrukturierung von keratinischen Fasern, insbesondere menschlichen Haaren.

Alternativ kann die Zubereitung auch auf das Haut und/oder das Haar aufgebracht und dort bis zur nächsten Haut- bzw. Haarwäsche belassen werden. Ein weiterer Gegenstand der vorliegenden Erfindung ist daher ein Verfahren zur Behandlung von Haut oder Haar, bei dem eine erfindungsgemäße Zubereitung auf die Haut und/oder das Haar aufgetragen und dort bis zur nächsten Wäsche belassen wird.

Bevorzugte Verfahren der letztgenannten Art sind dadurch gekennzeichnet, daß die nächste Wäsche länger als 24 Stunden nach dem Auftragen der erfindungsgemäßen Zubereitung auf die Haut und/oder das Haar erfolgt.

**Patentansprüche**

1. Kit, umfassend

a) festigendes Haarbehandlungsmittel in fester Form, welches aufweist:

- mindestens 1,0 Gew.% mindestens eines festigenden Polymers,
- mindestens 0,1 Gew.% eines Auflösungsbeschleunigers und welches in Form eines Granulats oder als fester Block vorliegt,

b) Spendersystem, mit Hilfe dessen das Haarbehandlungsmittel angewendet wird und das aus einer Mühle oder Reibe besteht, mit deren Hilfe von dem festen Block oder von dem Granulat ein Pulver abgerieben wird.

**2.** Verfahren zur Behandlung von Haar, bei dem ein festigendes Haarbehandlungsmittel in fester Form, welches aufweist:

- mindestens 1,0 Gew.% mindestens eines festigenden Polymers,
- mindestens 0,1 Gew.% eines Auflösungsbeschleunigers und welches in Form eines Granulats oder als fester Block vorliegt, mit Hilfe eines Spendersystems, das aus einer Mühle oder Reibe besteht mit deren Hilfe von dem festen Block oder von dem Granulat ein Pulver abgerieben wird, auf das Haar aufgetragen wird.

**Claims**

**1.** Kit comprising

a) a fixing hair treatment agent in solid form, which includes:

- at least 1.0% by weight of at least one fixing polymer,
- at least 0.1 % by weight of a dissolution accelerator,

and which is present in the form of a granulate or as a solid block,
b) a dispenser system by means of which the hair treatment product is used and which is made up of a mill or grater by means of which a powder is rubbed off from the solid block or from the granulate.

**2.** Method for treating hair, in which a fixing hair treatment agent in solid form, which includes:

- at least 1.0% by weight of at least one fixing polymer,
- at least 0.1% by weight of a dissolution accelerator,

and which is present in the form of a granulate or as a solid block,
is applied to the hair by means of a dispenser system which is made up of a mill or grater by means of which a powder is rubbed off from the solid block or from the granulate.

**Revendications**

**1.** Kit comprenant

a) un agent de traitement capillaire fixant sous forme solide, qui comporte :

- au moins 1,0% en poids d'au moins un polymère fixant,
- au moins 0,1% en poids d'un accélérateur de dissolution et

qui existe sous la forme d'un granulat ou d'un bloc solide,
b) un système distributeur qui permet d'appliquer l'agent de traitement capillaire et qui est constitué d'un broyeur ou d'une râpe permettant d'obtenir une poudre à partir du bloc solide ou du granulat.

**2.** Procédé de traitement capillaire dans lequel on applique sur les cheveux un agent de traitement capillaire fixant sous forme solide qui comporte

- au moins 1,0% en poids d'au moins un polymère fixant,
- au moins 0,1% en poids d'un accélérateur de dissolution et
qui existe sous la forme d'un granulat ou d'un bloc solide, à l'aide d'un système distributeur qui est constitué d'un broyeur ou d'une râpe permettant d'obtenir une poudre à partir du bloc solide ou du granulat.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10312270 A
- WO 9840462 A, Rettenmaier
- WO 9855583 A
- WO 9855590 A, Unilever
- WO 9840463 A
- DE 19709991
- DE 19710254, Henkel

**In der Beschreibung aufgeführte Nicht-Patentliteratur**

- *J.Pharm.Sci.,* 1972, 61
- Römpp Chemielexikon. vol. 6, 4440
- **VOIGT.** Lehrbuch der pharmazeutischen Technologie. 1987, 182-184